# EUROPEAN PATENT SPECIFICATION

(11) **EP 0 752 886 B1**
(45) Date of publication and mention of the grant of the patent: **28.01.1998**
(21) Application number: 94926106.9
(22) Date of filing: 25.08.1994
(51) Int. Cl.: A61K 39/00, A61K 39/385, C07K 19/00

(54) **INDUCING ANTIBODY RESPONSE AGAINST SELF-PROTEINS WITH THE AID OF FOREIGN T-CELL EPITOPES**
DIE INDUKTION DER ANTIKÖRPERANTWORT GEGEN EIGENE PROTEINE MIT HILFE FREMDER T-ZELLEPITOPE
PROCEDE D'INDUCTION DE REACTIONS IMMUNITAIRES CONTRE LES PROTEINES ENDOGENES A L'AIDE D'EPITOPES DE LYMPHOCYTES T EXOGENES

(30) Priority: 26.08.1993 DK 96493
(43) Date of publication of application: 15.01.1997
(73) Proprietor: M&E BIOTECH A/S, 2970 Horsholm (DK)
(72) Inventor: MOURITSEN, Soren, DK-3460 Birkerod (DK); ELSNER, Henrik, DK-2700 Bronshoj (DK)
(74) Representative: Christiansen, Ejvind
(86) International application number: DK9400318
(87) International publication number: WO9505849

(56) References cited:
- EP-A- 269 455
- EP-A- 343 460
- WO-A-83/03971
- WO-A-88/07869
- WO-A-89/12458
- WO-A-90/10707
- WO-A-90/15627
- WO-A-91/01330
- WO-A-91/02005
- WO-A-92/18150
- WO-A-92/19746
- WO-A-93/05810
- WO-A-93/23076
- US-A- 4 684 623
- US-A- 4 772 685
- NATIONAL LIBRARY OF MEDICINE DATABASE, MEDLINE, File Med 93, NLM accession no. 93013892, SAD S. et al., "Bypass of Carrier-Induced Epitope-Specific Suppression Using a T-Helper Epitope"; & IMMUNOLOGY 1992 Aug; 76(4): 599-603.
- DIALOG INFORMATION SERVICES, File 154, Medline accession no. 08270666, Medline accession no. 92408666, LOWENADLER B. et al.: "T and B Cell Responses to Chimeric Proteins Containing Heterologous T Helper Epitopes Inserted at Different Positions"; & MOL. IMMUNOL. Oct 1992, 29(10) p1185-90.
- METHODS IN ENZYMOLOGY, Volume 178, 1989, M.J. FRANCIS et al., "Peptide Vaccines Based on Enhanced Immunogenicity of Peptide Epitopes Presented with T-Cell Determinants or Hepatitis B Core Protein", page 659 - page 676.
- SCIENCE, Volume 249, July 1990, H.M. ETLINGER et al., "Use of Prior Vaccinations for the Development of New Vaccines", page 423 - page 425.
- DIALOG INFORMATION SERVICES, File 154, Medline, Dialog accession no. 07947371, Medline accession no. 92085371, SCHODEL F. et al.: "The Position of Heterologous Epitopes Inserted in Hepatitis B Virus Core Particles Determines their Immunogenicity Published Erratum Appears in J Virol 1992 Jun"; 66(6):3977; & J VIROL (UNITED STATES) Jan 1992, 66 (1) p106-14.
- DIALOG INFORMATION SERVICES, File 154, Medline, Dialog accession no. 07446388, Medline accession no. 90353388, LOWENADLER B. et al.: "Enhanced Immunogenicity of Recombinant Peptide Fusions Containing Multiple Copies of a Heterologous T Helper Epitope"; & EUR. J. IMMUNOL., Jul 1990, 20 (7) p1541-5.
- NATIONAL LIBRARY OF MEDICINE (NLM), File Medline, Medline accession no. 88131024, MURPHY J.R. et al.: "Interleukin 2 Toxin: a Step Toward Selective Immunomodulation"; & AM J KIDNEY DIS 1988 Feb;11(2):159-62.

## Description

### Field of the invention

This invention concerns a novel method for utilizing the immune apparatus to remove and/or down-regulate self-proteins, the presence of which somehow is unwanted in the individual. These could be proteins which are causing disease and/or other undesirable symptoms or signs of disease. Such proteins are removed by circulating autoantibodies which specifically are induced by vaccination. This invention describes a method for developing such autovaccines.

### Background of the invention

Physiologically, the vertebrate immune system serves as a defense mechanism against invasion of the body by infectious objects such as micro-organisms. Foreign proteins are effectively removed via the reticuloendothelial system by highly specific circulating antibodies, and viruses and bacteria are attacked by a complex battery of cellular and humoral mechanisms including antibodies, cytotoxic T lymphocytes, Natural Killer cells, complement etc. The leader of this battle is the T helper (T_{H}) lymphocyte which, in collaboration with the Antigen Presenting Cells (APC), regulate the immune defense via a complex network of cytokines.

T_{H} lymphocytes recognize protein antigens presented on the surface of the APC. They do not recognize, however, native antigen per se. Instead, they appear to recognize a complex ligand consisting of two components, a "processed" (fragmented) protein antigen (the so-called T cell epitope) and a Major Histocompatibility Complex class II molecule (O. Werdelin et al., Imm. Rev. 106, 181 (1988)). This recognition eventually enables the T_{H} lymphocyte specifically to help B lymphocytes to produce specific antibodies towards the intact protein antigen (Werdelin et al., supra). A given T cell only recognizes a certain antigen-MHC combination and will not recognize the same or another antigen presented by a gene product of another MHC allele. This phenomenon is called MHC restriction.

Normally the individual's own proteins (the so-called self- or autoproteins) are not attacked by the immune apparatus. The described events thus generally are beneficial to the individual, but in rare cases the process goes wrong, and the immune system turns towards the individual's own components, which may lead to an autoimmune disease.

The presence of some self-proteins is inexpedient in situations where they, in elevated levels, induce disease symptoms. High levels of immunoglobulins of the IgE class are e.g. known to be important for the induction of type I allergy, and tumor necrosis factor I (TNFα) is known to be able to cause cachexia in cancer patients and patients suffering from other chronic diseases (H.N. Langstein et al., Cancer Res. 51, 2302-2306, 1991). TNFα also plays important roles in the inflammatory process (W.P. Arend et al., Arthritis Rheum. 33, 305-315, 1990). Hormones in sex-hormone dependent cancer are other examples of proteins which are unwanted in certain situations. There is therefore a need for the provision of a method for the development of autovaccines against such self-proteins.

Fragments of self-proteins are also presented by the APC, but normally such fragments are ignored or not recognized by the T helper lymphocytes. This is the main reason why individuals generally do not harbor autoantibodies in their serum.

It is, however, possible artificially to induce antibodies against self-proteins. This can be done, as previously mentioned, by covalent conjugation of the self-protein to an appropriate large foreign carrier protein as e.g. tetanus toxoid or key-hole limpet hemocyanin (KLH). Talwar et al. (G.P. Talwar et al, Int. J. Immunopharmacol. 14, 511-514, 1992) have been able to prevent reproduction in women using a vaccine consisting of a conjugate of human chorionic gonadotropin and tetanus toxoid. There are also other examples of such autoimmunogenic conjugates which have been used therapeutically in man and in animal models (D.R. Stanworth et al., Lancet 336, 1279-1281 (1990)). During the processing of such conjugates in the APC, the necessary T_{H} lymphocyte stimulatory epitopes are provided from the foreign protein eventually leading to the induction of antibodies against the self-protein as well as against the carrier protein. One disadvantage of using this principle is, however, that the antibody response towards the self-protein will be limited due to shielding of epitopes by the covalently linked carrier protein. Another disadvantage is the increased risk of inducing allergic side-effects due to the contemporary induction of an undesired very strong antibody response against the foreign carrier protein.

Other researchers have conjugated a single peptide predicted to be a T cell epitope chemically as a carrier to a self-peptide [D-Lys⁶]GnRH which is a decapeptide acting as a hapten and managed to induce an autoantibody response with MHC restriction to that particular T cell epitope (S. Sad et al., Immunology 76, 599-603, 1992). This method seems to be more effective compared with conjugation to large carrier proteins. However, it will only induce antibodies in a population expressing the appropriate MHC molecules. This means that a rather large number of different T cell epitopes has to be conjugated to the self-peptide which will eventually disturb the B cell epitopes on the surface of the self-peptide. Extensive conjugation of proteins may furthermore have the opposite effect with regard to immunogenicity (international patent application No. WO 87/00056) and the surface exposed peptide T cell epitopes may be destroyed by proteolytic enzymes during antigen processing (S. Mouritsen, Scand. J. Immunol. 30, 723, 1989), making that method inexpedient. Also, the exact structure of such multi-conjugated self-peptides will not be chemically and pharmaceutically well-defined.

Recently an improved method has been proposed for breaking the B cell autotolerance by chemical conjugation of B and optionally also peptide T cell epitopes to a high molecular weight dextran molecule (WO 93/23076 published November 25, 1993). The disadvantages mentioned above, however, also hold true for said method.

Löwenadler et al, (Medline Accession No. 9208571 & Mol Immunol 29 1992 1185-1190; discloses chimeric proteins containing one or more copies of heterologous T helper epitopes, in particular ovalbumin 323-339 (ova), inserted at different positions. The chimeric proteins comprise one or more ova sequences flanked by the heat-stable enterotoxin of E. coli and the nine C-terminal amino acids of human insulin-like growth factor 1 (IGF-1), together with a synthetic analogue of the IgG-binding domain of protein A, the latter to aid the purification by means of IgG-Sepharone ™ affinity chromatography. Thus the chimeric proteins are fusion constructs of several different proteins. The effect of the presence of the ova sequences on the production of antibodies to the enterotoxin and the IGF-1 peptide was investigated in mice, i.e. the proteins used are not self-proteins.

WO-A-93/05810, Hellman, discusses possible mutations of protein and it is suggested that "heavily mutated" forms of the CH2-CH3 domains of IgE (a self-protein) may be used in a vaccine, these being created by exchange, deletion or insertion of amino acids in the sequence. This would create T-cell epitopes within the amino acid sequence, allowing the CH2-CH3 domains to act as an antigen per se and thus obviating the need to couple the protein to a carrier. "Slightly mutated" forms, according to the disclosure of Hellman, still need to be coupled to a suitable carrier protein in order to act as an antigen.

It has been suggested previously that a universally recognized strong T cell epitope could be associated with a foreign peptide having an antigenic structure representing a B-cell epitope using recombinant DNA technology (EP-A2-0 343 460) It has also been suggested to use peptidyl resin conjugates comprising an immunogenic or antigenic peptide incorporating a helper T-cell (T_{H} lymphocyte) reactive epitope and preferably a B-cell reactive epitope in the preparation of immunogenic compositions, e.g. vaccines. The conjugates are prepared by solid phase synthesis, preferably on a polyamide resin. (WO 90/15627). While the intent is to increase an antibody response towards the peptides in question, it has not been proposed that this could be done with the purpose of breaking the autotolerance of the immune system and induce an antibody response against self-proteins. Using these methods for induction of autoantibodies against self-proteins one a priori would expect the same rules to be true with regard to the above-mentioned limitations of the MHC restriction of the response. Surprisingly, however, by modulation of self-proteins using the method according to the invention, wherein a self-protein analog is produced by substitution of one or more peptide fragments by a corresponding number of peptides known to contain immunodominant T-cell epitopes, said substitution being carried out so as to essentially preserve the overall tertiary structure of the original self-protein, it proved possible to induce an equally fast and even a stronger autoantibody response against TNFα despite the fact that the inserted T cell epitope used was not restricted to the MHC molecules of the immunized mice, vide Example 3 below. The reason for this observation is not clear but may be due to the appearance of new MHC binding segments in the mutagenized area in the self-protein. However, the experiment shown in example 5 demonstrates that this is probably not the case, since synthetic peptides representing overlapping regions of the implanted ovalbumin T cell epitope in ubiquitin did not bind strongly to any of the MHC class II molecules of the H-2^{k} mice in which this recombinant molecule was highly immunogenic.

Most of the potential MHC class II binding segments of a protein are normally cryptic and will not be presented to the host T cells by the antigen presenting cells (S. Mouritsen et al, Scand. J. Immunol. 34, 421, 1991). The observed lacking correspondence between the MHC restriction of the inserted T cell epitope and the restriction of the antibody response could perhaps be due to a general disturbance of the intra-molecular competition of binding to MHC molecules by different self-protein segments. According to the method of the invention non-tolerized cryptic self-protein segments may be presented to the T cells leading to breaking of the T cell as well as the B cell autotolerance towards the protein. In accordance with the invention and illustrated in all the examples described below, a fragment of the self-protein was substituted with a foreign T cell epitope. This deletion followed by a substitution with an other protein fragment minimally obscure the tertiary structure of the self-proteins, but may also contribute strongly to the disturbance of said intramolecular competition of the MHC class II binding self-segments. This concept is therefore clearly different from the above-mentioned prior art mechanisms and methods. Independently of the operating mechanism of action by the method according to the invention, it is more technically advantageous compared to the known methods for breaking the B cell autotolerance, since it is possible to induce antibodies in a broad population of MHC molecules by insertion of a minimal number of different foreign T cell epitopes.

The present invention thus is based on the surprising fact that injection of recombinant self-proteins, which have been appropriately modulated by deletion of one or more peptide fragments and simultaneous insertion of a corresponding number of foreign T cell epitopes, so as to produce a self-protein analog with an essentially preserved tertiary structure, induces a profound autoantibody response against the unmodified self-proteins. Surprisingly the MHC-restriction of the auto-antibody response induced was not necessarily confined to that of the inserted T cell epitope. By inducing minimal tertiary structural changes in the highly conserved self-protein ubiquitin, as well as in TNFα, foreign T cell epitopes having a length of 12-15 amino acids were inserted using genetic engineering methods. These recombinant self-protein analogs were purified, emulsified in adjuvant and injected into mice. Within only one week an autoantibody response against ubiquitin could be detected in serum from these mice. Non-modified, recombinant ubiquitin treated and injected in the same way was not able to induce a response.

By using this principle for developing vaccines against undesirable proteins, the risk of inducing allergic side-effect is reduced, and toxic self-proteins such as TNFα can simultaneously be detoxified by removing or mutating biologically active protein segments. The epitope-shielding effect described above is not a problem, and autoantibodies against ubiquitin were induced much faster as compared to the known technique, in which the self-protein is conjugated to a carrier protein or peptide. Importantly, by this method it furthermore seems possible to temporarily break the autotolerance of the T cells as well as that of the B cells of the individual, and such recombinant proteins will be self-immunogenic in a large population expressing many different MHC class II molecules.

The vaccine according to the invention consists of one or more self-protein analogs modulated as described above and formulated with suitable adjuvants, such as calcium phosphate, saponin, quil A or biodegradable polymers. The modulated self-protein analogs may be prepared as fusion proteins with suitable, immunologically active cytokines, such as GM-CSF or interleukin 2.

The autovaccine may i.a. be a vaccine against TNFα or γ-interferon for the treatment of patients with cachexia, e.g. cancer patients, or a vaccine against IgE for the treatment of patients with allergy. Furthermore, it may be a vaccine against TNFα, TNFβ or interleukin 1 for the treatment of patients with chronic inflammatory diseases.

The invention is illustrated in the following examples:

### EXAMPLE 1

### Substitution of foreign T cell epitopes into ubiquitin

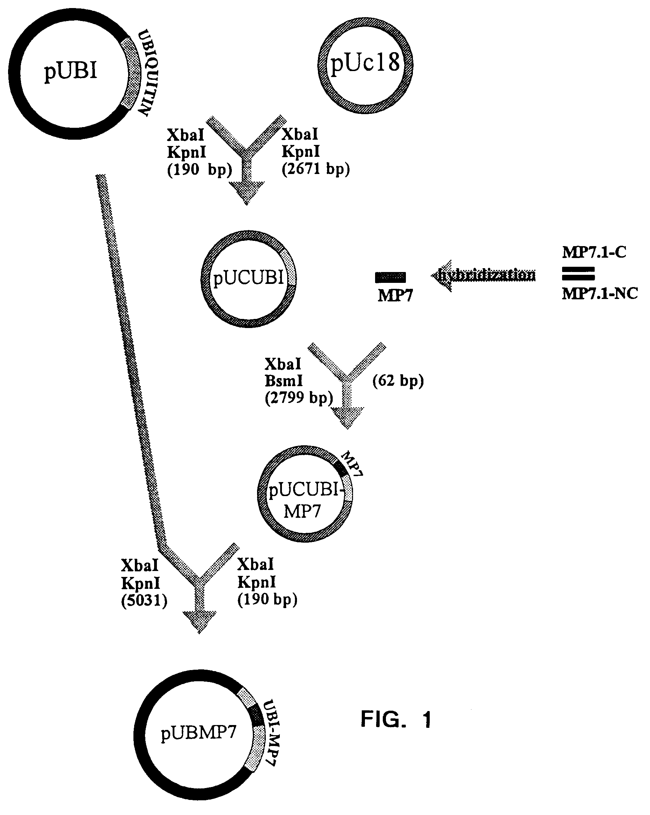

An overview of this procedure is shown in fig. 1 using the T cell epitope MP7 as example. The gene sequences representing MP7 (MP7.1-C and MP7.1-NC) were synthesized as two complementary oligonucleotides designed with appropriate restriction enzyme cloning sites. The amino acid sequence of MP7 is PELFEALQKLFKHAY, Mouritsen et al, Scand. J. Immunol. 30, 723-730, 1989. The oligonucleotides were synthesized using conventional, automatic solid phase oligonucleotide synthesis and purified using agarose gel electrophoresis and low melting agarose. The desired bands were cut out from the gels, and known quantities of oligonucleotides were mixed, heated to 5C below their theoretical melting point (usually to approximately 65C) for 1-2 hours, and slowly cooled to 37C. At this temperature the hybridized oligonucleotides were ligated to the vector fragments containing part of the ubiquitin gene. The subsequent analysis of positive clones using restriction fragment analysis and DNA sequencing was done by conventional methods ("Molecular Cloning", Eds.: T. Maniatis et al. 2 ed. CSH Laboratory Press, 1989).

### EXAMPLE 2

### Induction of autoantibodies against ubiquitin by vaccination with modified ubiquitin analogs

Genes containing sequences encoding the foreign T cell epitopes OVA (325-336) from ovalbumin (OVA) and HEL (50-61) from hen eggwhite lysozyme (HEL), respectively, were expressed in E. coli strain, AR58 under control of the heat sensitive S repressor regulated promotor. Expression of the recombinant ubiquitin proteins were verified using a polyclonal anti-ubiquitin antibody and Western-blotting ("Antibodies", Eds.: D. Harlow et al., CSH Laboratory Press, 1988). The recombinant proteins were purified using conventional methods (Maniatis et al., supra).

Mice were injected i.p. with 100 µg of ubiquitin or its analogs in phosphate buffered saline (PBS) emulsified in Freunds Complete adjuvant. Booster injections of the same amount of antigen emulsified 1:1 in Freunds Incomplete adjuvant were performed i.p. at days 14 and 28. Five Balb/c mice in each group were examined and blood samples were examined for the presence of anti-ubiquitin antibodies on day 7, 14, 21, 28, 35, and 42 using conventional ELISA methodology.

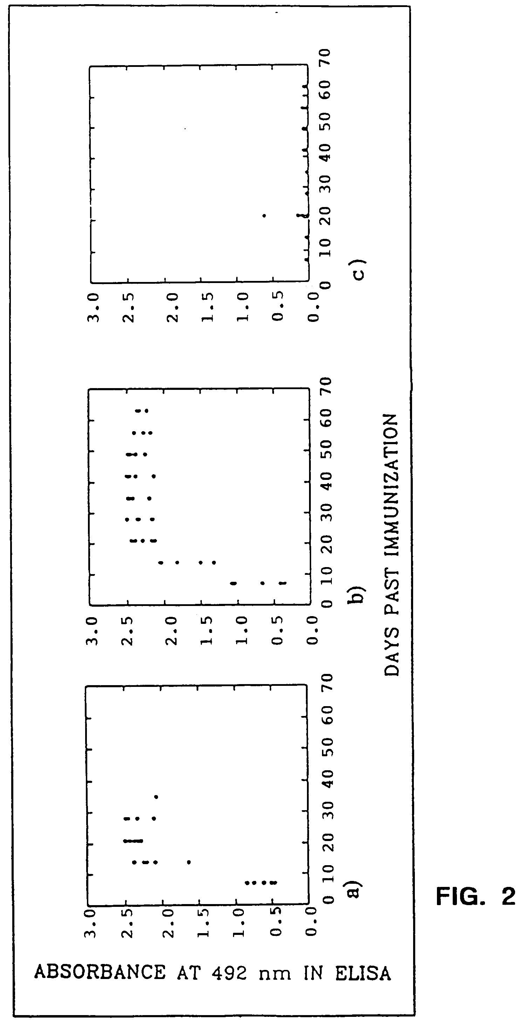

The results exemplified by the antibody response against two different ubiquitin analogs containing the T cell epitopes OVA(325-336) and HEL(50-61), respectively, are shown in fig. 2. The amino acid sequence of the inserted OVA(325-336) epitope is: QAVHAAHAEINE and the amino acid sequence of the HEL(50-61) epitope is STDYGILQINSR.

A clear antibody response against native ubiquitin could be detected within only one week from the first injection of antigen reaching a maximum within 2 weeks. Antiubiquitin antibodies produced in rabbits by covalently conjugating ubiquitin to bovine immunoglobulin reached maximum values after a much longer immunization period (data not shown).

### EXAMPLE 3

### Induction of autoantibodies against tumor necrosis factors (TNF) by vaccination with appropriately modified TNF analogs

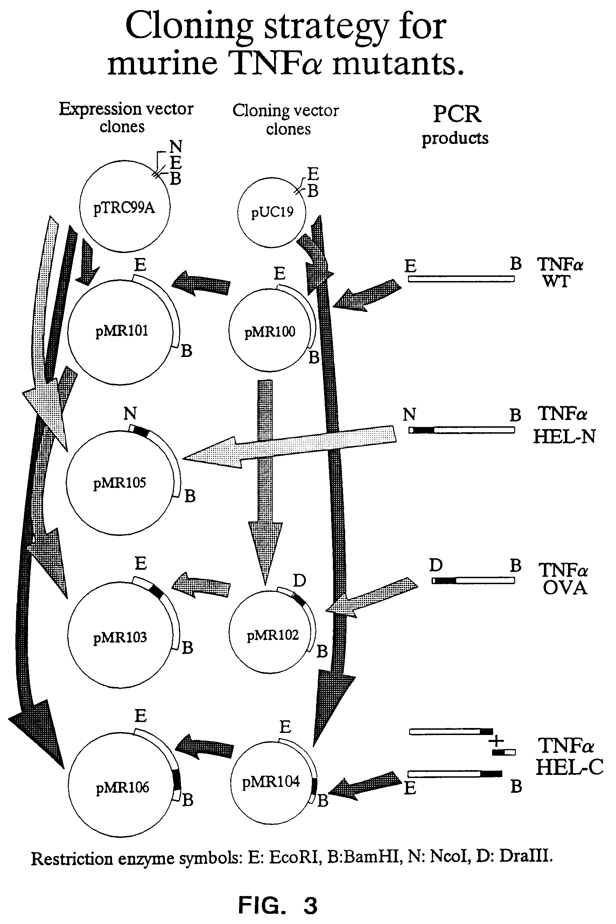

The gene coding for the structural part of the native murine TNFα protein (MR101) was obtained by Polymerase Chain Reaction (PCR) cloning of the DNA. In the MR103 TNFα analog the ovalbumin (OVA) H-2^{d} restricted T cell epitope sequence 325-334 (QAVHAAHAET) replaces the amino acids 26-35 in the cloned TNFα sequence, a substitution of an amphiphatic α-helix. Substitutions in this region of the TNFα detoxifies the recombinant protein (X. Van Ostade et al., Nature 361, 266-269, 1993). In the MR105 analog the H-2^{k} restricted T cell epitope from hen eggwhite lysozyme (HEL), amino acid sequence 81-96 (SALLSSDITASVNCAK) replaces the amino acids 5-20 in the cloned TNFα sequence. In the MR106 TNFα mutant the same epitope, amino acid sequence 81-95 (SALLSSDITASVNCA) replaces the amino acids 126-140 in the cloned TNFα sequence. The genetic constructions are shown in Fig. 3. Different techniques compared to the technique described in example 1 were used for exchanging parts of the TNFα gene with DNA coding for T cell epitopes. The MR105 and 106 constructs were made by introducing the mutant sequence by PCR recloning a part of the TNFα gene flanking the intended site for introducing the T cell epitope. The mutant oligonucleotide primer contained both a DNA sequence homologous to the TNFα DNA sequence as well as a DNA sequence encoding the T cell epitope. The PCR recloned part of the TNFα gene was subsequently cut with appropriate restriction enzymes and cloned into the MR101 gene. The MR103 construction was made by a modification of the "splicing by overlap extension" PCR technique (R. M. Horton et al., Gene 77, 61, 1989). Here two PCR products are produced, each covering a part of the TNFα gene, and additionally each PCR product contains half of the T cell epitope sequence. The complete mutant TNFα gene was subsequently made by combining the two PCR products in a second PCR. Finally, the complete genetic constructions were inserted into protein expression vectors. Subsequently, all genetic constructions were analyzed by restriction fragment analysis and DNA sequencing using conventional methods ("Molecular Cloning", Eds,: T. Maniatis et al. 2.ed. CSH Laboratory Press, 1989). The recombinant proteins were expressed in E.coli and purified by conventional protein purification methods.

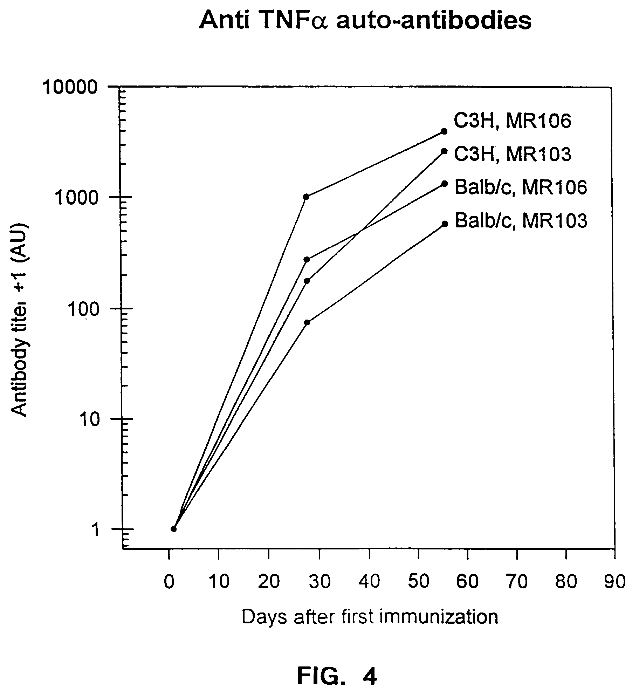

Groups of BALB/c (MHC haplotype H-2^{d}) and C3H (MHC haplotype H-2^{k}) mice, respectively, were immunized subcutaneously with 100 Tg of semi-purified MR103 and MR106 emulsified in Freunds' complete adjuvant. Every second week the immunizations were repeated using incomplete Freunds' adjuvant. All mice developed an early and strong antibody response against biologically active MR101. This was measured by a direct ELISA method using passively adsorbed 100% pure MR101 (Fig. 4). Control mice immunized with MR101 and PBS, respectively, showed no antibody reactivity towards MR101.

Strikingly, the antibody response towards MR101 was not MHC restricted corresponding to the implanted T cell epitopes, since both mice strains of different MHC haplotypes responded well to MR103 and MR106 containing differently restricted T-cell epitopes (Fig. 4). Taken together these results illustrate (a) the ability of the self-protein analogs produced by the method according to the invention to induce autoantibodies towards a secreted autoprotein and (b) the improved efficiency of the herein described method with regard to inducing a response in a broader MHC population than predicted by the MHC binding ability of the inserted T cell epitopes. The immune response against MR101 induced by the recombinant self-protein analogs MR103 and MR106 was much stronger and more high-titered compared to the immune response induced by aldehyde conjugated MR101 (see Example 4).

### EXAMPLE 4

### Induction of autoantibodies against TNFα by self-protein analogs produced by the method according to the invention compared to unmodified self-protein conjugated to E. coli proteins.

The induction of autoantibodies against TNFα by the method of the present invention has been directly compared to the autoantibody response induced when using a conjugate of TNFα and E. coli proteins, which must contain small single T cell epitope peptides as well as larger foreign carrier proteins.

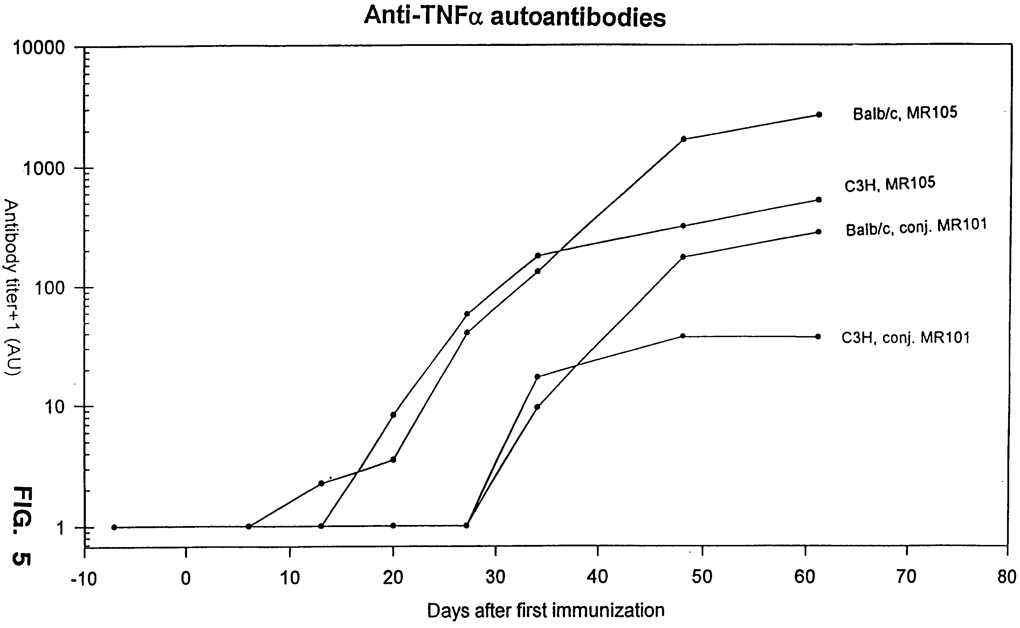

Semi-purified recombinant murine TNFα (MR101) was conjugated to E. coli proteins in PBS, pH 7.4, using 0.5% formaldehyde. Conjugation of the proteins was confirmed by SDS-PAGE. These conjugates were subsequently used for immunization of C3H mice. Another group of C3H mice was vaccinated with semi-purified non-conjugated self-protein analog MR105. About 100 µg of recombinant TNFα analog and conjugate were emulsified 1:1 in Freunds' complete adjuvant and injected subcutaneously in each group of mice. MR105 is biologically inactive as judged by the L929 bioassay for TNFα. In subsequent immunizations every second week incomplete Freunds' adjuvant was used. Both groups eventually developed autoantibodies against highly purified biologically active MR101 as determined by ELISA, but the immune response against the non-conjugated analog MR105 produced by the method of the invention was induced earlier and was of a higher titer (Fig. 5).

### EXAMPLE 5

### The possible MHC class II binding of peptides representing overlapping sequences of self-protein as well as of the ovalbumin T cell epitope inserted in ubiquitin.

Peptide-MHC complexes were obtained by incubating ¹²⁵I-labeled peptide (10-100 nM) with affinity purified MHC class II molecules (2-10 µM) at room temperature for 3 days (S. Mouritsen, J. Immunol. 148, 1438-1444, 1992). The following peptides were used as radiolabeled markers of binding: Hb(64-76)Y which binds strongly to the E^{k} molecule and HEL(46-61)Y which binds strongly to the A^{k} molecule. These complexes were co-incubated with large amounts of cold (non-radiolabeled) peptide (> 550 Tm) which is sufficient to inhibit totally all immunologically relevant MHC class II binding. Either the same peptides were used, or three different overlapping peptides were used, said peptides representing the flanking regions as well as the entire OVA(325-336) T cell epitope which was substituted into ubiquitin (see Example 2). The three peptides were: TITLEVEPSQAVHAA (U(12-26)), PSQAVHAAHAEINEKE (U(19-34)) and HAEINEKEGIPPDQQ (U(27-41)). The reaction buffer contained 8 mM citrate, 17 mM phosphate, and 0.05% NP-40 (pH 5) and peptide-MHC class II complexes were separated (in duplicate) from free peptide by gel filtration using G25 spun columns. Both the radioactivities of the excluded "void" volume and of the included volume were measured by gamma spectrometry. The competitive inhibition of maximal binding (in percent) by addition of cold peptide was calculated. The results are shown in Table I.

**TABLE I**

| Peptide/MHC | Hb(64-76) | HEL(46-61) | U(12-26) | U(19-34) | U(27-41) |
|---|---|---|---|---|---|
| A^{k} | 28.6 | 97.4 | 35.3 | 44.6 | 7.8 |
| E^{k} | 92.6 | 0.0 | 45.6 | 12.2 | 0.0 |

It can be seen that total inhibition of the binding of the radiolabeled peptides Hb(64-76)Y and HEL(46-61)Y to E^{k} and A^{k} respectively could only be achieved using cold versions of the same peptides. Although some inhibition of binding was seen by U(12-26) and U(19-34) using these extreme amounts of cold peptide, it is likely that the affinity of these peptides to the H-2^{k} MHC class II molecules is very low. Therefore this seems not to be sufficient to explain the strong immunogenicity in the H-2^{k} mouse strain of the ubiquitin analog containing the ovalbumin T cell epitope. More likely, other and previously non-tolerized self-epitopes are presented to the T cell in these animals.

### EXAMPLE 6

### Treatment of diabetes or inflammatory disease by vaccination with appropriately modified TNFα analogs

Genes coding for TNFα are modified by insertion of appropriate gene segments coding for T cell epitopes derived from e.g. tetanus toxin or influenza hemagglutinin. Such genes are expressed in appropriate expression vectors in e.g. E. coli or insect cells. The recombinant TNFα proteins were purified using conventional methods ("Molecular Cloning", Eds.: T. Maniatis et al. 2. ed. CSH Laboratory Press, 1989).

Optionally such recombinant proteins can be coupled to immunologically active cytokines such a GMCSF or interleukin 2.

The recombinant proteins can be formulated with appropriate adjuvants and administered as an anti-TNFα vaccine to patients suffering from diseases where TNFα is important for the pathogenesis. The induced anti-TNFα antibodies will thereby affect the diseases.

One example of said diseases is the chronic inflammatory diseases such as e.g. rheumatoid arthritis where TNFα is believed to play an important role (reviewed in: F.M. Brennan et al., Br. J. Rheumatol. 31, 293-298, 1992). TNFα is also believed to play an important role in the cachec tic conditions seen in cancer and in chronic infectious diseases such as AIDS (reviewed in M. Odeh. J. Intern. Med. 228, 549-556, 1990). It is also known that TNF participates in septic shock (reviewed in: B.P. Giroir, Crit. Care. Med., 21, 780-789, 1993). Furthermore, it has been shown that TNFα may play a pathogenetic role in the development of type II diabetes mellitus (CH Lang et al., Endocrinology 130, 43-52, 1992).

### LEGENDS TO FIGURES

Fig. 1. Schematic overview of the cloning strategy used in the construction of a ubiquitin gene with an implanted foreign T cell epitope (MP7). Restriction enzyme digestions, hybridization and ligation procedures are indicated with arrows. Fragment sizes are shown in parentheses.

Fig. 2. Reactivity toward immobilized bovine ubiquitin in sera from mice immunized with recombinant ubiquitin and analogs containing the implanted T cell epitopes OVA(325-336) and HEL (50-61), respectively. Fig. 2a) sera from Balb/c mice immunized with recombinant ubiquitin containing OVA(325-336). Fig. 2b) sera from Balb/c mice immunized with recombinant ubiquitin containing the T cell epitope HEL(50-61). Fig. 2c) sera from Balb/c mice immunized with recombinant non-modified ubiquitin. Sera (diluted 1:100) were tested in a standard ELISA assay using non-modified bovine ubiquitin immobilized on the solid phase.

Fig. 3. Schematic overview of the cloning strategy used in the construction of the recombinant TNFα mutants. PCR products and restriction enzyme digestions are indicated.

Fig. 4. Induction of TNFα autoantibodies by vaccination of Balb/c or C3H mice with semipurified MR103 and MR106. The antibody titers were measured by ELISA and expressed as arbitrary units (AU) referring to a strong standard anti-serum from one mouse. The plotted values represent a mean titer for 5 animals. Freunds complete adjuvant was used as adjuvant for the first immunization. All subsequent immunizations at 14 days intervals were done with Freunds incomplete adjuvant. Mice immunized in parallel with native MR101 in PBS did not develop detectable TNFα autoantibodies (data not shown). Non-detectable antibody titers were assigned the titer value 1.

Fig. 5. Anti TNFα autoantibodies induced by vaccination with non-conjugated MR105 and MR101 conjugated to E. coli proteins, respectively. C3H mice and Balb/c mice were immunized with both preparations. The immunizations, measurements and calculations of mean antibody titers were done as described in example 4.

## Claims

1. A method for the modification of self-proteins so as to induce antibody response against the unmodified self-proteins following administration of said modified self-proteins to the host, CHARACTERIZED by providing a self-protein analog by molecular biological means by substitution of one or more peptide fragments of the self-protein by a corresponding number of peptides known to contain immunodominant foreign T cell epitopes, said substitution being carried out so as to essentially preserve the overall tertiary structure of the original self-protein.

2. A method according to claim 1, wherein said immunodominant foreign T-cell epitopes are inserted so as to preserve flanking regions from the original self-protein on both sides of the T-cell epitope.

3. A method according to any of claims 1 or 2, wherein the immunodominant T cell epitope(s) originate(s) from tetanus toxoid or diphtheria toxoid.

4. An autovaccine against undesirable self-proteins in humans or animals, CHARACTERIZED in that it comprises one or more self-protein analogs modified according to any of claims 1 - 3 and formulated with pharmaceutically acceptable adjuvants, such as calcium phosphate, saponin, quil A and biodegradable polymers.

5. An autovaccine according to claim 4, CHARACTERIZED in that the self-protein analog is present in the form of a fusion protein with suitable, immunologically active cytokines, such as GM-CSF or interleukin 2.

6. An autovaccine according to claim 4, CHARACTERIZED in that it is a vaccine against TNFα or γ-interferon for the treatment of patients susceptible to cachexia, e.g. cancer patients.

7. An autovaccine according to claim 4, CHARACTERIZED in that it is a vaccine against IgE for the treatment of patients with allergy.

8. An autovaccine according to claim 4, CHARACTERIZED in that it is a vaccine against TNFα, TNFβ or interleukin 1 for the treatment of patients with chronic inflammatory diseases.

9. An autovaccine according to claim 8, CHARACTERIZED in that it is a vaccine for treatment of patients with rheumatoid arthritis or an inflammatory bowel disease.

10. An autovaccine according to claim 4 or 5, CHARACTERIZED in that it is a vaccine against TNFα for the treatment of diabetes mellitus.

## Patentansprüche

1. Verfahren zur Modifizierung von Eigenproteinen, so daß diese eine Antikörperantwort gegen nicht-modifizierte Eigenproteine nach Verabreichung der modifizierten Eigenproteine an den Wirt induzieren,
**gekennzeichnet** durch Bereitstellung eines Eigenproteinanalogs mittels Molekularbiologie durch Substitution von einem oder mehrerer Peptidfragmente des Eigenproteins durch eine entsprechende Anzahl von Peptiden, von denen man weiß, daß sie immundominante Fremd-T-Zell-Epitope enthalten, wobei die Substitution so durchgeführt wird, daß im wesentlichen die Gesamt-Tertiärstruktur des ursprünglichen Eigenproteins erhalten bleibt.

2. Verfahren nach Anspruch 1, worin die immundominanten Fremd-T-Zell-Epitope so eingefügt werden, daß die flankierenden Regionen aus dem ursprünglichen Eigenprotein an beiden Seiten des T-Zell-Epitops erhalten bleiben.

3. Verfahren nach einem der Ansprüche 1 oder 2, worin das (die) immundominante(n) T-Zell-Epitop(e) aus Tetanustoxoid oder Diphtherietoxoid stammt (en).

4. Autoimpfstoff gegen unerwünschte Eigenproteine in Menschen oder Tieren, **gekennzeichnet** darin, daß es ein oder mehrere Eigenproteine, modifiziert nach einem der Ansprüche 1 bis 3, enthält und mit pharmazeutische verträglichen Adjuvanzien, wie Kalziumphosphat, Saponin, Quil A und bioabbaubaren Polymeren formuliert ist.

5. Autoimpfstoff nach Anspruch 4, dadurch **gekennzeichnet,** daß das Eigenproteinanalog in Form eines Fusionsproteins mit geeigneten, immunologisch aktiven Cytokinen, wie GM-CSF oder Interleukin 2, vorliegt.

6. Autoimpfstoff nach Anspruch 4, dadurch **gekennzeichnet,** daß es ein Impfstoff gegen TNFα oder γ-Interferon zur Behandlung von Patienten ist, die für Cachexie anfällig sind, z. B. Krebspatienten.

7. Autoimpfstoff nach Anspruch 4, dadurch **gekennzeichnet,** daß es ein Impfstoff gegen IgE zur Behandlung von Patienten mit Allergie ist.

8. Autoimpfstoff nach Anspruch 4, dadurch **gekennzeichnet,** daß es ein Impfstoff gegen TNFα, TNFβ oder Interleukin 1 zur Behandlung von Patienten mit chronischen Entzündungskrankheiten ist.

9. Autoimpfstoff nach Anspruch 8, dadurch **gekennzeichnet,** daß es ein Impfstoff zur Behandlung von Patienten mit rheumatoider Arthritis oder entzündlicher Darmerkrankung ist.

10. Autoimpfstoff nach Anspruch 4 oder 5, dadurch **gekennzeichnet,** daß es ein Impfstoff gegen TNFα zur Behandlung von Diabetes mellitus ist.

## Revendications

1. Procédé de modification de protéines du soi de manière à induire une réponse anticorps contre les protéines du soi non modifiées après administration chez l'hôte desdites protéines du soi modifiées, caractérisé en ce que l'on fournit un analogue de la protéine du soi par des moyens biologiques par substitution d'un ou plusieurs fragments peptidiques de la protéine du soi par un nombre correspondant de peptides connus pour contenir des épitopes immunodominants de cellules T exogènes, ladite substitution étant effectuée de manière à essentiellement préserver la structure tertiaire globale de la protéine du soi d'origine.

2. Procédé selon la revendication 1, dans lequel lesdits épitopes immunodominants de cellules T exogènes sont insérés de manière à préserver les régions bordantes de la protéine du soi d'origine sur les deux côtés de l'épitope de la cellule T.

3. Procédé selon la revendication 1 ou 2, dans lequel le (ou les) épitope(s) immunodominants de cellules T proviennent de l'anatoxine tétanique ou de l'anatoxine diphtérique.

4. Autovaccin contre les protéines du soi indésirables chez l'homme ou l'animal, caractérisé en ce qu'il comprend un ou plusieurs analogues modifiés d'une protéine du soi selon l'une quelconque des revendications 1 a 3, et formulés avec des adjuvants pharmaceutiquement acceptables, tels que le phosphate de calcium, saponine, quil A et les polymères biodégradables.

5. Autovaccin selon la revendication 4, caractérisé en ce que l'analogue de la protéine du soi est présente sous la forme d'une protéine de fusion avec des cytokines immunologiquement actives appropriées, tels que GM-CSF ou l'interleukine 2.

6. Autovaccin selon la revendication 4, caractérisé en ce qu'il représente un vaccin contre TNFα ou l'interféron-γ pour le traitement de patients atteints de cachexie, par exemple des patient atteints d'un cancer.

7. Autovaccin selon la revendication 4, caractérisé en ce qu'il représente un vaccin contre les IgE pour le traitement des patients allergiques.

8. Autovaccin selon la revendication 4, caractérisé en ce qu'il représente un vaccin contre TNFα, TNFβ, ou l'interleukine 1, pour le traitement de patients atteints de maladies inflammatoires chroniques.

9. Autovaccin selon la revendication 8, caractérisé en ce qu'il représente un vaccin pour le traitement de patients atteints de rhumatisme articulaire ou de maladie inflammatoire intestinale.

10. Autovaccin selon la revendication 4 ou 5, caractérisé en ce qu'il représente un vaccin contre TNFα pour le traitement des diabètes sucrés.
